# EUROPEAN PATENT APPLICATION

(11) **EP 4 830 777 A1**
(43) Date of publication of application: **28.10.2026**
(21) Application number: 26173601.1
(22) Date of filing: 21.04.2026
(51) Int. Cl.: A61N 5/10

(54) **RADIATION TREATMENT PLAN OPTIMIZATION METHOD AND APPARATUS**

(30) Priority: 22.04.2025 US 202519186318
(71) Applicant: Siemens Healthineers International AG, 6312 Steinhausen (CH)
(72) Inventor: TALLINEN, Tuomas, 00100 Helsinki (FI); PELTOLA, Jarkko, 00100 Helsinki (FI)
(74) Representative: Mathisen & Macara LLP

(57) **Abstract**

When optimizing a radiation treatment plan for a particular patient using a particular radiation treatment apparatus, and particularly when the approach to optimizing comprises direct aperture optimization, by one approach, a control circuit can determine (201) whether to apply field-specific asymmetry as a function of at least a first criterion (202) and when, upon determining to apply field-specific asymmetry, apply field-specific asymmetry when establishing multi-leaf collimator apertures relative to a target projection (204).

## Description

### TECHNICAL FIELD

These teachings relate generally to treating a patient planning target volume with energy pursuant to an energy-based treatment plan and more particularly to optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

With typical prior art direct aperture optimization approaches, setting up treatment fields and their field-apertures - including a collimator angle and the rectangular field size defined by X1, X2, Y1, Y2 jaw positions (and/or multi-collimator aperture sizing) - typically requires user expertise in order to achieve a good final plan quality. Achieving a useful result can become more problematic when the target volume size exceeds the maximum available leaf modulation range of one field.

In many typical prior art direct aperture optimization approaches, leaf-aperture optimization is typically based only on independent two-dimensional target fluence maps. In particular, such optimization occurs without utilizing knowledge about the number of arc fields, their gantry rotation ranges, and/or collimator angles. The corresponding results can be non-optimal for cases with single or multi-field arc setups. As a result, an iterative fine-tuning of the field-apertures in each arc field setup may be needed to improve plan quality for a given patient model geometry.

### SUMMARY OF THE INVENTION

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Direct aperture optimization is one approach to such optimization. Direct aperture optimization integrates the optimization of fluence intensities and leaf positions of a multi-leaf collimator in a single iterative process. This approach can provide for directly tailoring the physical parameters of radiation delivery to a desired dose distribution.

The present teachings can provide for creating an initial leaf configuration per field for use in iterative direct aperture optimization. The initial leaf aperture configuration for each field can be created so that the configuration depends on the total arc field setup. This approach can be based upon defining a field-specific asymmetry in the X (leaf) direction when positioning/guiding the initial leaf-apertures relative to a target projection. These initial apertures can be modified from the apertures fitted to full target projections (called target fitted apertures here) by first reducing the aperture opening width by a desired modulation factor, and then offsetting the result inside the target-fitted apertures.

The present invention provides a radiation treatment plan optimization method as set out in the claims. The present invention further provides a radiation treatment plan optimization apparatus as set out in the claims. The present invention further provides non-transitory computer-readable medium for use when optimizing a radiation treatment plan for a particular patient using a particular radiation treatment apparatus as set out in the claims. A radiation treatment plan optimization apparatus of the present invention comprises a radiation treatment apparatus having a control circuit, which control circuit may be is configured to carry out one or more of the steps, actions, and/or functions required in embodiments of the present invention. The control circuit may be operably coupled to a memory. The memory may be integral to the control circuit or can be physically discrete therefrom, in whole or in party. The memory may store optimization information for a particular patient and information regarding a particular radiation treatment platform. The control circuit may be operably coupled to a user interface and/or a network interface. The control circuit may be operably coupled to an imaging apparatus. The control circuit is suitably configured to output an optimized energy-based treatment plan, such as an optimized radiation treatment plan. The radiation apparatus suitably includes at least an energy source and may also include one or more of a beam shaping apparatus, a patient support apparatus, a patient fixation apparatus, a gantry. The control circuit may be configured to control operation of the radiation source and/or beam shaping apparatus. The control circuit may be configured to control movement of the radiation source, the gantry, the patient support apparatus and the beam shaping apparatus.

Generally speaking, these various embodiments can be employed when optimizing a radiation treatment plan for a particular patient using a particular radiation treatment apparatus, and particularly when the approach to optimizing comprises direct aperture optimization. By one approach, a control circuit can determine whether to apply field-specific asymmetry as a function of at least a first criterion and when, upon determining to apply field-specific asymmetry, apply field-specific asymmetry when establishing multi-leaf collimator apertures relative to a target projection.

And as used herein, "field-specific asymmetry" will be understood to refer to biasing a beam-shaping aperture sideways with respect to a target volume projection.

These teachings will accommodate any of a variety of criteria to be applied in the foregoing regards. By one approach, for example, the first criterion can comprise an arc length. As another example, the first criterion can comprise a multi-leaf collimator angle. These teachings may comprise making the aforementioned determination as a function of at least two criteria. As one illustrative example, this determination can be made as a function of a first criterion that comprises an arc length and a second criterion that comprises a multi-leaf collimator angle.

By one approach, the aforementioned determination can comprise determining to apply field-specific asymmetry when a radiation treatment apparatus arc length exceeds 180 degrees, a multi-leaf collimator planar angle is less than 30 degrees, and a corresponding field aperture size fully encompasses a target volume instead of being a split field that encompasses only a part of the target volume.

By one approach, in lieu of the foregoing or in combination therewith, the aforementioned determination can comprise determining whether to apply field-specific asymmetry as a function, at least in part, of whether the radiation treatment plan corresponds to a double-field treatment arrangement. If desired, this determination can further comprise determining whether to apply field-specific asymmetry as a further function of whether a corresponding field aperture size fully encompasses a target volume instead of being a split field that encompasses only a part of the target volume.

By one approach, the aforementioned application of field-specific asymmetry when establishing initial multi-leaf collimator apertures relative to a target projection can comprise modifying an original aperture that is fitted to a full target projection by reducing a corresponding original aperture opening width by a modulation factor and then offsetting the reduced aperture opening inside the original target aperture. By one approach, in lieu of the foregoing or in combination therewith, the aforementioned application of field-specific asymmetry when establishing multi-leaf collimator apertures relative to a target projection can comprise applying field-specific asymmetry when establishing multi-leaf collimator apertures relative to a target projection on a field-by-field basis.

By one approach, these teachings can comprise a non-transitory computer-readable medium having a computer program that itself comprises instructions that, when the computer program is executed by a computer, causes the computer to carry out one or more of the actions, steps, or functions described herein, including determining whether to apply field-specific asymmetry as a function of at least a first criterion and when, upon determining to apply field-specific asymmetry, applying field-specific asymmetry when establishing multi-leaf collimator apertures relative to a target projection. For example, the control circuit can apply field-specific asymmetry when establishing multi-leaf collimator apertures relative to a target projection.

So configured, these teachings can serve, for example, to reduce manual tuning work, avoid suboptimal plans, and increase modulation capabilities of multi-arc plans.

These and other benefits may become clearer upon making a thorough review and study of the following detailed description.

### BRIEF DESCRIPTION OF DRAWINGS

Various needs are at least partially met through provision of the radiation treatment plan optimization method and apparatus described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a schematic representation as configured in accordance with various embodiments of these teachings;
FIG. 4 comprises a schematic representation as configured in accordance with various embodiments of these teachings;
FIG. 5 comprises a schematic representation as configured in accordance with various embodiments of these teachings;
FIG. 6 comprises a decision tree as configured in accordance with various embodiments of these teachings; and
FIG. 7 comprises a graph as configured in accordance with various embodiments of the invention.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

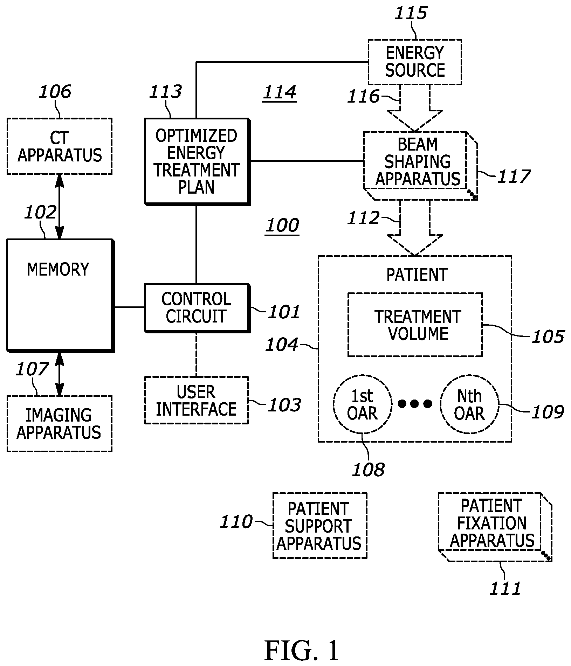

Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM)).

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

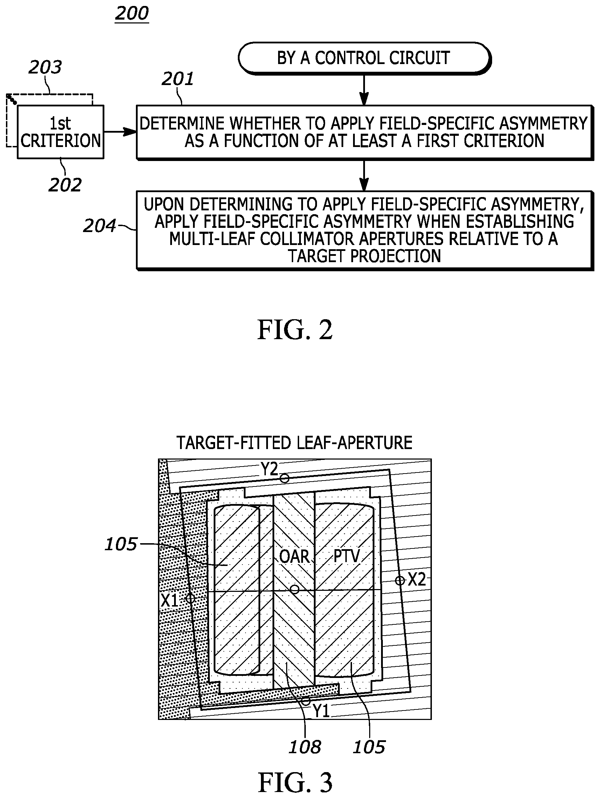

Referring now to FIG. 2, a process 200 that can be carried out, for example, in conjunction with the above-described application setting (and more particularly via the aforementioned control circuit 101) will be described. Generally speaking, this process 200 can serve to facilitate generating an optimized radiation treatment plan 113 to thereby facilitate treating a particular patient 104 with therapeutic radiation 112 using a particular radiation treatment platform 114 per that optimized radiation treatment plan 113.

In this illustrative example, this process 200 can be used when optimizing a radiation treatment plan for a particular patient 104 using a particular radiation treatment apparatus 114.

At block 201, the control circuit 101 determines whether to apply field-specific asymmetry as a function of at least a first criterion. A "field" refers to a specific area or volume of the patient's body that is targeted and to exposed to a controlled dose of radiation. A field is often defined by the spatial coordinates and geometric parameters that outline the area to be treated, including the size, shape, and angle of the radiation beam's entry. By one approach, fields can be shaped by various means, such as using multi-leaf collimators, custom blocks, or other beam-shaping devices. Many radiation treatment plans provide for multiple fields to accommodate different angles from which the radiation beam is aimed at the target volume.

These teachings will also accommodate determining whether to apply field-specific asymmetry as a function of at least a first and a second criterion (such as, for example, the aforementioned arc length and multi-leaf collimator angle).

As an illustrative example regarding the aforementioned determination, determining whether to apply field-specific asymmetry as a function of at least a first criterion can comprise determining to apply field-specific asymmetry when a radiation treatment apparatus's arc length exceeds 180°, a multi-leaf collimator planar angle is less than 30°, and a corresponding field aperture size fully encompasses a target volume instead of being a split field that encompasses only a part of the target volume.

As another illustrative example in these regards, determining whether to apply field-specific asymmetry as a function of at least a first criterion can comprise determining whether to apply field-specific asymmetry as a function, at least in part, of whether the radiation treatment plan corresponds to a double-field treatment arrangement. By one approach, the foregoing can comprise determining whether to apply field-specific asymmetry as a further function of whether a corresponding field aperture size fully encompasses a target volume instead of being a split field that encompasses only a part of the target volume.

At block 204, upon determining to apply field-specific asymmetry, the control circuit 101 can apply field-specific asymmetry when establishing multi-leaf collimator apertures relative to a target projection. For example, the control circuit 101 can modify an original aperture that is fitted to a full target projection by reducing a corresponding original aperture opening width by a modulation factor and then offsetting the reduced aperture opening inside the original target aperture. By one approach, the control circuit 101 can apply field-specific asymmetry when establishing multi-leaf collimator apertures relative to a target projection on a field- by- field basis.

These teachings will accommodate, if desired, then using the resultant optimized radiation treatment plan to administer therapeutic radiation to the particular patient using the particular radiation treatment apparatus.

Further details that comport with these teachings will now be presented. It will be understood that the specific details of these examples are intended to serve an illustrative purpose and are not intended to suggest any particular limitations with respect to these teachings.

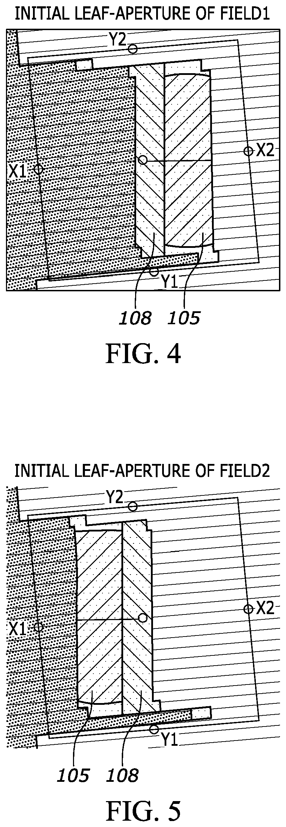

FIGS. 3-5 illustrate an example in the foregoing regards for asymmetric leaf-aperture initializations for a double-field scenario. FIG. 3 illustrates a patient treatment volume 105 that appears, in this field of view, on either side of an organ-at-risk 108. FIG. 4 presents a Field1 that is initialized to the right side of the target volume 105 projection while FIG. 5 presents a Field2 that is initialized to the left side of the target volume 105 projection.

By one approach, target projections can be limited to given field-apertures of each field. During direct aperture optimization iterations, leaves can be moved anywhere inside the field-apertures. Relative initial leaf-aperture positions can be defined per field and applied equally to all control points of the field. (Control points refer to specific positions or angles at which a radiation beam is turned on or off during treatment. Control points typically serve to help shape the radiation beam and to optimize the dose distribution to target the tumor while sparing surrounding healthy tissues.)

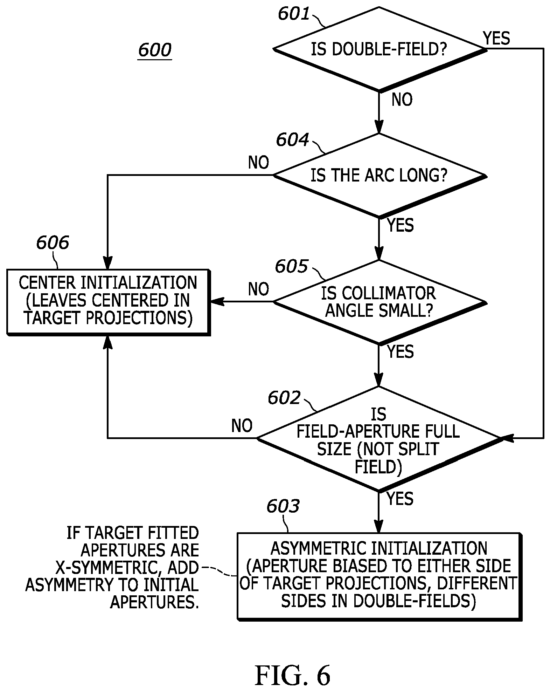

FIG. 6 illustrates one approach for finding the relevant asymmetries via a decision tree 600.

At block 601, a decision is made regarding whether the plan presents a double-field. When a plan includes a pair of arcs having a common arc span, couch angle, and isocenter, and similar field-apertures (defined by collimator angle and an X1, X2, Y1, Y2 rectangle), they can be identified as a double-field. In this case, their leaf-apertures can be initialized to the left (a first arc) and to the right (a second arc). Accordingly, the multi-leaf collimator leaf movement directions align in both fields and the X-range where they are allowed to move are the same, and only the initial leaf-positions are different.

When the foregoing is true, at block 602 a decision is made regarding whether the field-aperture is full size (and hence not a split field). If double-field setups have a left/right division already in their field-apertures, this decision can essentially quantify that circumstance. Any additional left/right leaf-positioning can be applied only as needed via block 603 using asymmetric initialization where the aperture can be biased to either side of corresponding target projections (and typically using both sides in a double-field scenario).

When the double-field decision at block 601 yields a not-true result, the applicant has determined that for arcs other than double-fields, asymmetrically positioned leaf-apertures can be a good starting point for iterative direct aperture optimization to produce high target dose homogeneity and to avoid possible critical organs or normal tissue at the isocenter in bilateral cases. But for asymmetric apertures to work as desired in such a setting, the arc should be long (for example, at least half-arc or more) and the collimator angle should be small, for the beam to sweep through the whole target volume during gantry rotation. (In this application setting, "small" can be, for example, less than 10 degrees of angle, less than 20 degrees of angle, less than 30 degrees of angle, less than 40 degrees of angle, or any value with that range as desired.) These conditions are checked by the "Is the arc long" decision block 604 and the "is collimator angle small" 605 blocks in the decision tree 600. When these conditions are not met, the leaves can be initialized to the center of the target projection(s) instead as shown at block 606. Arc length and collimator angle can also be quantified continuously and normalized between [0, 1], and the amount asymmetry can be a continuous function of the arc length and collimator angle, if desired.

When the foregoing decisions determine a double-field situation, or that the arc is long and the collimator angle is small, a decision can be made as described above at block 602 regarding whether the field-aperture is full size (and that this is not a split field). If untrue, center initialization at block 606 can proceed. If true, asymmetric initialization at block 603 can proceed.

If desired, these teachings will further accommodate having the leaf positioning asymmetry be scaled by how X-symmetric on average the target-fitted leaf-apertures are. The more X-symmetric the target-fitted leaf-apertures are, the more asymmetric the leaf-positioning can be to counter that. If the target-fitted leaf-apertures are already sufficiently asymmetric, for example, if the field-aperture clips one side of the target volume, there can be less or no need for additional asymmetry from leaf-positioning.

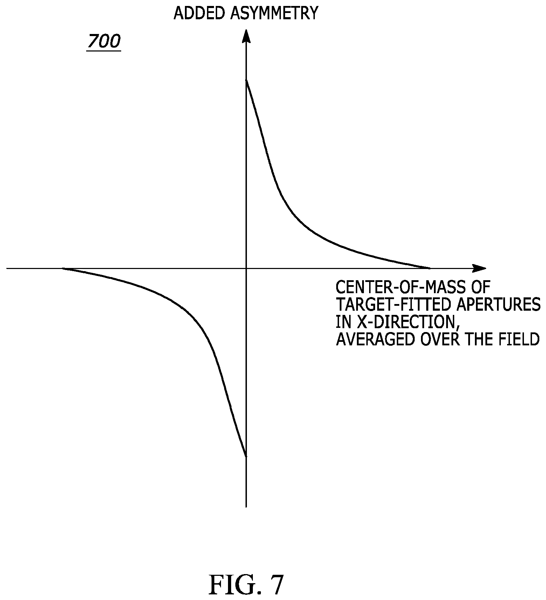

FIG. 7 presents a graph that illustrates the amount of added asymmetry with respect to the center-of-mass of target-fitted apertures in the X-direction (averaged over the field). This graph presents a continuous form graphical elaboration for the rule/decision discussed above for block 602.

These teachings can provide a number of benefits. As a first example, these approaches can reduce manual tuning work that can be needed to identify and determine optimal field-apertures. As a second example, these teachings can help to prevent suboptimal plans that may arise from using less useful field-apertures that are difficult for current iterative direct aperture optimization techniques (for example, double-fields with identical field-apertures, and X-symmetric field-apertures on cases where the optimal plan is clearly X-asymmetric). And as a third example, these teachings can help to increase the modulation capabilities of multi-arc plans by incorporating coordination between fields in the direct aperture optimization step. This can be relevant especially on radiation treatment machines whose field-apertures can cover whole target volume in most cases.

Further aspects of these teachings are provided by the subject matter of the following clauses (where it will be understood that any of these clauses can be combined with any one of more of the other clauses as desired).

Clause 1. A method for use when optimizing a radiation treatment plan for a particular patient using a particular radiation treatment apparatus, the method comprising: by a control circuit: determining whether to apply field-specific asymmetry as a function of at least a first criterion; upon determining to apply field-specific asymmetry, applying field-specific asymmetry when establishing multi-leaf collimator apertures relative to a target projection.

Clause 2. The method of clause 1 wherein the first criterion comprises one of: an arc length; a multi-leaf collimator angle.

Clause 3. The method of clause 1 wherein determining whether to apply field-specific asymmetry as a function of at least a first criterion comprises determining whether to apply field-specific asymmetry as a function of at least a first and a second criterion.

Clause 4. The method of clause 3 wherein the first criterion comprises an arc length and the second criterion comprises a multi-leaf collimator angle.

Clause 5. The method of clause 1 wherein determining whether to apply field-specific asymmetry as a function of at least a first criterion comprises determining to apply field-specific asymmetry when a radiation treatment apparatus arc length exceeds 180 degrees, a multi-leaf collimator planar angle is less than 30 degrees, and a corresponding field aperture size fully encompasses a target volume instead of being a split field that encompasses only a part of the target volume.

Clause 6. The method of clause 1 wherein applying field-specific asymmetry when establishing initial multi-leaf collimator apertures relative to a target projection comprises modifying an original aperture that is fitted to a full target projection by reducing a corresponding original aperture opening width by a modulation factor and then offsetting the reduced aperture opening inside the original target aperture.

Clause 7. The method of clause 1 wherein optimizing the radiation treatment plan comprises using direct aperture optimization.

Clause 8. The method of clause 1 wherein applying field-specific asymmetry when establishing multi-leaf collimator apertures relative to a target projection comprises applying field-specific asymmetry when establishing multi-leaf collimator apertures relative to a target projection on a field-by-field basis.

Clause 9. The method of clause 1 wherein determining whether to apply field-specific asymmetry as a function of at least a first criterion comprises determining whether to apply field-specific asymmetry as a function, at least in part, of whether the radiation treatment plan corresponds to a double-field treatment arrangement.

Clause 10. The method of clause 9 wherein determining whether to apply field-specific asymmetry further comprises determining whether to apply field-specific asymmetry as a further function of whether a corresponding field aperture size fully encompasses a target volume instead of being a split field that encompasses only a part of the target volume.

Clause 11. An apparatus for use when optimizing a radiation treatment plan for a particular patient using a particular radiation treatment apparatus, the apparatus comprising: a control circuit configured to: determine whether to apply field-specific asymmetry as a function of at least a first criterion; upon determining to apply field-specific asymmetry, apply field-specific asymmetry when establishing multi-leaf collimator apertures relative to a target projection.

Clause 12. The apparatus of clause 11 wherein the first criterion comprises one of: an arc length; a multi-leaf collimator angle.

Clause 13. The apparatus of clause 11 wherein the control circuit is configured to determine whether to apply field-specific asymmetry as a function of at least a first criterion by determining whether to apply field-specific asymmetry as a function of at least a first and a second criterion.

Clause 14. The apparatus of clause 13 wherein the first criterion comprises an arc length and the second criterion comprises a multi-leaf collimator angle.

Clause 15. The apparatus of clause 11 wherein the control circuit is configured to determine whether to apply field-specific asymmetry as a function of at least a first criterion by determining to apply field-specific asymmetry when a radiation treatment apparatus arc length exceeds 180 degrees, a multi-leaf collimator planar angle is less than 30 degrees, and a corresponding field aperture size fully encompasses a target volume instead of being a split field that encompasses only a part of the target volume.

Clause 16. The apparatus of clause 11 wherein the control circuit is configured to apply field-specific asymmetry when establishing multi-leaf collimator apertures relative to a target projection by modifying an original aperture that is fitted to a full target projection by reducing a corresponding original aperture opening width by a modulation factor and then offsetting the reduced aperture opening inside the original target aperture.

Clause 17. The apparatus of clause 11 wherein the control circuit is configured to apply field-specific asymmetry when establishing multi-leaf collimator apertures relative to a target projection by applying field-specific asymmetry when establishing multi-leaf collimator apertures relative to a target projection on a field-by-field basis.

Clause 18. The apparatus of clause 11 wherein the control circuit is configured to determine whether to apply field-specific asymmetry as a function of at least a first criterion by determining whether to apply field-specific asymmetry as a function, at least in part, of whether the radiation treatment plan corresponds to a double-field treatment arrangement.

Clause 19. The apparatus of clause 18 wherein the control circuit is further configured to determine whether to apply field-specific asymmetry by determining whether to apply field-specific asymmetry as a further function of whether a corresponding field aperture size fully encompasses a target volume instead of being a split field that encompasses only a part of the target volume.

Clause 20. A non-transitory computer-readable medium for use when optimizing a radiation treatment plan for a particular patient using a particular radiation treatment apparatus comprising instructions stored thereon, that when executed on a processor, perform the steps of: determining whether to apply field-specific asymmetry as a function of at least a first criterion; upon determining to apply field-specific asymmetry, applying field-specific asymmetry when establishing multi-leaf collimator apertures relative to a target projection.

Those skilled in the art will recognize that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A radiation treatment plan optimization method (200) for a particular patient, the method comprising:
by a control circuit (101):
determining (201) whether to apply field-specific asymmetry as a function of at least a first criterion (202);
upon determining to apply field-specific asymmetry, applying field-specific asymmetry when establishing multi-leaf collimator apertures relative to a target projection (204)
wherein, optionally, the first criterion comprises one of:
an arc length;
a multi-leaf collimator angle.

2. The method of claim 1 wherein determining (201) whether to apply field-specific asymmetry as a function of at least a first criterion comprises determining whether to apply field-specific asymmetry as a function of at least a first and a second criterion (202, 203), and optionally the first criterion comprises an arc length and the second criterion comprises a multi-leaf collimator angle.

3. The method of claim 1 or 2 wherein determining (201) whether to apply field-specific asymmetry as a function of at least a first criterion comprises determining to apply field-specific asymmetry when a radiation treatment apparatus arc length exceeds 180 degrees, a multi-leaf collimator planar angle is less than 30 degrees, and a corresponding field aperture size fully encompasses a target volume instead of being a split field that encompasses only a part of the target volume.

4. The method of any one of the claims 1 to 3 wherein applying field-specific asymmetry when establishing initial multi-leaf collimator apertures relative to a target projection (204) comprises
modifying an original aperture that is fitted to a full target projection by reducing a corresponding original aperture opening width by a modulation factor and then offsetting the reduced aperture opening inside the original target aperture, and/or
applying field-specific asymmetry when establishing multi-leaf collimator apertures relative to a target projection on a field-by-field basis.

5. The method of any one of the preceding claims wherein optimizing the radiation treatment plan comprises using direct aperture optimization (204).

6. The method of any one of the preceding claims wherein determining whether to apply field-specific asymmetry as a function of at least a first criterion comprises determining whether to apply field-specific asymmetry as a function, at least in part, of whether the radiation treatment plan corresponds to a double-field treatment arrangement.

7. The method of claim 6 wherein determining whether to apply field-specific asymmetry further comprises determining whether to apply field-specific asymmetry as a further function of whether a corresponding field aperture size fully encompasses a target volume instead of being a split field that encompasses only a part of the target volume.

8. A radiation treatment plan optimization apparatus for a particular patient comprises a radiation treatment apparatus (114), the apparatus comprising:
a control circuit (101) configured to:
determine (201) whether to apply field-specific asymmetry as a function of at least a first criterion (202);
upon determining to apply field-specific asymmetry, apply field-specific asymmetry when establishing multi-leaf collimator apertures relative to a target projection (204)
wherein, optionally, the first criterion comprises one of:
an arc length;
a multi-leaf collimator angle.

9. The apparatus of claim 8 wherein the control circuit is configured to determine whether to apply field-specific asymmetry as a function of at least a first criterion by determining whether to apply field-specific asymmetry as a function of at least a first and a second criterion (202, 203) and optionally the first criterion comprises an arc length and the second criterion comprises a multi-leaf collimator angle.

10. The apparatus of claim 8 or 9 wherein the control circuit is configured to determine whether to apply field-specific asymmetry as a function of at least a first criterion by determining to apply field-specific asymmetry when a radiation treatment apparatus arc length exceeds 180 degrees, a multi-leaf collimator planar angle is less than 30 degrees, and a corresponding field aperture size fully encompasses a target volume instead of being a split field that encompasses only a part of the target volume.

11. The apparatus of any one of claims 8 to 10 wherein the control circuit is configured to apply field-specific asymmetry when establishing multi-leaf collimator apertures relative to a target projection by modifying an original aperture that is fitted to a full target projection by reducing a corresponding original aperture opening width by a modulation factor and then offsetting the reduced aperture opening inside the original target aperture.

12. The apparatus of any one of claims 8 to 11 wherein the control circuit is configured to apply field-specific asymmetry when establishing multi-leaf collimator apertures relative to a target projection by applying field-specific asymmetry when establishing multi-leaf collimator apertures relative to a target projection on a field-by-field basis.

13. The apparatus of any one or claims 8 to 12 wherein the control circuit is configured to determine whether to apply field-specific asymmetry as a function of at least a first criterion by determining whether to apply field-specific asymmetry as a function, at least in part, of whether the radiation treatment plan corresponds to a double-field treatment arrangement.

14. The apparatus of any one of claims 8 to 13 wherein the control circuit is further configured to determine whether to apply field-specific asymmetry by determining whether to apply field-specific asymmetry as a further function of whether a corresponding field aperture size fully encompasses a target volume instead of being a split field that encompasses only a part of the target volume.

15. A non-transitory computer-readable medium for use when optimizing a radiation treatment plan for a particular patient using a particular radiation treatment apparatus (114) comprising instructions stored thereon, that when executed on a processor, perform the steps of:
determining (201) whether to apply field-specific asymmetry as a function of at least a first criterion (202);
upon determining to apply field-specific asymmetry, applying field-specific asymmetry when establishing multi-leaf collimator apertures relative to a target projection (204).
